# EUROPÄISCHE PATENTANMELDUNG

(11) **EP 4 477 641 A1**
(43) Veröffentlichungstag der Anmeldung: **18.12.2024**
(21) Anmeldenummer: 23179203.7
(22) Anmeldetag: 14.06.2023
(51) Int. Cl.: C07C 31/125, C07C 29/149, C07C 67/38, C07C 69/24, C07C 7/04, C07C 11/02

(54) **VERFAHREN ZUR HERSTELLUNG EINES ALKOHOLS DURCH ALKOXYCARBONYLIERUNG VON DI-ISOBUTEN MIT VORHERIGER DESTILLATION UND ANSCHLIESSENDER HYDRIERUNG**

(71) Anmelder: Evonik Oxeno GmbH & Co. KG, 45772 Marl (DE)
(72) Erfinder: KUCMIERCZYK, Peter, 44628 Herne (DE); FRANKE, Robert, 45772 Marl (DE); SALE, Anna Chiara, 45657 Recklinghausen (DE); FRIDAG, Dirk, 45721 Haltern am See (DE); MARKOVIC, Ana, 45721 Haltern am See (DE); RIX, Armin Matthias, 45770 Marl (DE); TERMÜHLEN, Maren, 44135 Dortmund (DE); HARDING, Laura-Selin, 46286 Dorsten (DE); METTERNICH, Jan Benedikt, 45657 Recklinghausen (DE)
(74) Vertreter: Evonik Patent Association

(57) **Zusammenfassung**

Gegenstand der Erfindung ist ein Verfahren zur Herstellung eines Zielalkohols durch Hydrieren eines Esters, der durch Alkoxycarbonylierung von Di-isobuten entsteht. Der bei der der Alkoxycarbonylierung eingesetzte Di-isobutenstrom wird dabei vor der Alkoxycarbonylierung einer Destillation unterworfen wird, um 2,4,4-Trimethylpent-1-en in dem zu alkoxycarbonylierenden Strom anzureichern. Die Alkoxycarbonylierung wird mit einem Alkohol und Kohlenmonoxid in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, durchgeführt.

## Beschreibung

Die vorliegende Erfindung betrifft ein Verfahren zur Herstellung eines Zielalkohols durch Hydrieren eines Esters, der durch Alkoxycarbonylierung von Di-isobuten entsteht. Der bei der der Alkoxycarbonylierung eingesetzte Di-isobutenstrom wird dabei vor der Alkoxycarbonylierung einer Destillation unterworfen wird, um 2,4,4-Trimethylpent-1-en in dem zu alkoxycarbonylierenden Strom anzureichern. Die Alkoxycarbonylierung wird mit einem Alkohol und Kohlenmonoxid in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, durchgeführt.

Die Herstellung von Alkoholen in der industriellen Chemie erfolgt zum größten Teil über die Hydroformylierung zur Herstellung eines Aldehyds mit nachfolgender Hydrierung des Aldehyds zum Alkohol. Wenngleich die Herstellung von Alkoholen im Wege der Hydroformylierung mit nachfolgender Hydrierung ein seit Jahrzehnten industriell etablierter und bewährter Prozess ist, weist er immer noch Verbesserungspotential auf. Ein Problem bei dieser Syntheseroute ist, dass bei der Hydroformylierung meistens hohe Drücke und hohe Temperaturen vorliegen und dadurch vergleichsweise hohe technische Anforderungen an die verwendeten Anlagen gestellt werden. Schließlich müssen die Anlagen die Drücke und Temperaturen aushalten können.

Di-isobuten ist ein technisch relevantes Erzeugnis, welches durch Dimerisierung von Isobuten gewonnen wird. Di-isobuten besteht aus den Isomeren 2,4,4-Trimethylpent-1-en (nachfolgend auch: TMP1) und 2,4,4-Trimethylpent-2-en (nachfolgend auch: TMP2) mit einer Massenverteilung TMP1 : TMP2 von etwa 78:22 bis 81 : 19 (Gleichgewichtsverteilung). Diese Mischung kann unter anderem in Carbonylierungsverfahren zu höherwertigen Produkten umgesetzt werden. Dabei weist speziell in Carbonylierungsverfahren, wie der Methoxycarbonylierung (Reaktionsprodukt ist hier der 3,5,5-Trimethylhesansäuremethylester) oder der Hydroformylierung (Reaktionsprodukt ist hier der 3,5,5-Trimethylhexanal) das innenständige Olefin TMP2 eine deutlich geringere Reaktivität als das endständige Olefin TMP1 auf. Oftmals können Reaktionsbedingungen oder Verweilzeiten bei diesen Carbonylierungsreaktionen aufgrund der mangelnden Stabilität der Katalysatoren oder aufgrund ökonomischer Faktoren (z. B. der Reaktorgröße) nicht dahingehend angepasst werden, dass das endständige TMP2 vollständig abreagiert.

Die Aufgabe der vorliegenden Erfindung war deshalb ein Verfahren zur Herstellung eines Zielalkohols, welches die vorgenannten Probleme nicht aufweist. Insbesondere sollte die Alkoxycarbonylierung mit Di-isobutenströmen, die einen hohen Anteil an 2,4,4-Trimethylpent-1-en aufweisen, durchgeführt werden. Weiterhin sollte ein alternativer Syntheseweg für die Herstellung eines Zielakohols erhalten werden, mit der sich die gewünschten Zielakohole relativ einfach erschließen lassen.

Diese Aufgabe konnte durch das erfindungsgemäße Verfahren nach dem unabhängigen Anspruch 1 gelöst werden. Bevorzugte Ausführungsformen sind in den abhängigen Ansprüchen angegeben.

Beschrieben wird vorliegend ein Verfahren zur Herstellung eines Zielalkohols, wobei das Verfahren zumindest die folgenden Schritte umfasst:
a. Zuführen eines Di-isobutenstroms, der 2,4,4-Trimethylpent-2-en und 2,4,4-Trimethylpent-1-en enthält, zu mindestens einer Destillationskolonne und Auftrennen des Di-isobutenstroms in mindestens einen gegenüber dem eingesetzten Di-isobutenstrom an 2,4,4-Trimethylpent-1-en angereicherten Kopfstrom, der mehr als 85 Gew.-% an 2,4,4-Trimethylpent-1-en enthält, und einen Reststrom, der gegenüber dem eingesetzten Di-isobutenstrom an 2,4,4-Trimethylpent-1-en abgereichert ist;
b. Zuführen des Kopfstroms zu einer Alkoxycarbonylierung, wodurch 2,4,4-Trimethylpent-2-en und 2,4,4-Trimethylpent-1-en mit einem Alkohol und Kohlenmonoxid in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, in einer Reaktionszone unter Erhalt eines flüssigen Produktgemisches umgesetzt werden, wobei das flüssige Produktgemisch zumindest den durch die Alkoxycarbonylierung gebildeten Ester, das homogene Katalysatorsystem, nicht umgesetztes 2,4,4-Trimethylpent-2-en und 2,4,4-Trimethylpent-1-en und nicht umgesetzten Alkohol umfasst;
c. Abtrennen des homogenen Katalysatorsystems aus dem flüssigen Produktgemisch unter Erhalt eines Rohproduktgemisches, welches zumindest den durch die Alkoxycarbonylierung gebildeten Ester, nicht umgesetztes 2,4,4-Trimethylpent-2-en und 2,4,4-Trimethylpent-1-en und nicht umgesetzte Alkohole umfasst;
d. Destillative Aufarbeitung des Rohproduktgemisches in mindestens einer Destillationskolonne zur Abtrennung der nicht umgesetzten Alkohole und das nicht umgesetzte 2,4,4-Trimethylpent-2-en und 2,4,4-Trimethylpent-1-en, wobei nicht umgesetzter Alkohol und das nicht umgesetzte 2,4,4-Trimethylpent-2-en und 2,4,4-Trimethylpent-1-en abgetrennt und zur Alkoxycarbonylierung in Schritt b zurückgeführt wird;
e. Hydrieren des in Schritt d erhaltenen Esters mit Wasserstoff in Gegenwart des heterogenen Katalysatorsystems in einer Hydrierzone unter Erhalt eines Alkoholgemisches, welches zumindest den Zielalkohol, den abgespaltenen Alkohol und nicht umgesetzte Ester umfasst; und
f. Abtrennen des in Schritt e gebildeten Zielalkohols in mindestens einem Trennverfahrensschritt, ausgewählt aus thermischer Trennung, beispielsweise Destillation, Extraktion, Kristallisation und Membrantrennung.

Ein Vorteil des Verfahrens ist die Destillation des Di-isobutenstroms vor der Akkoxycarbonylierung, weil dadurch der Anteil an 2,4,4-Trimethylpent-1-en im Kopfstrom der mindestens einen Destillationskolonne gegenüber dem eingesetzten Strom gesteigert werden kann. So ist es möglich Anteile von mehr als 85 Gew-%, vorzugsweise mehr als 90 Gew.-%, besonders bevorzugt mehr als 95 Gew.-% an 2,4,4-Trimethylpent-1-en im Strom zu erreichen, was für die nachgeschaltete Alkoxycarbonylierung aufgrund der höheren Reaktivität von 2,4,4-Trimethylpent-1-en vorteilhaft ist.

Schritt a des erfindungsgemäßen Verfahrens betrifft die Destillation eines Di-isobutenstroms, welcher 2,4,4-Trimethylpent-2-en und 2,4,4-Trimethylpent-1-en enthält. Der Di-isobutenstrom wird dabei in mindestens einen gegenüber dem eingesetzten Di-isobutenstrom an 2,4,4-Trimethylpent-1-en angereicherten Kopfstrom, der vorzugsweise mehr als 85 Gew.-% an 2,4,4-Trimethylpent-1-en, weiterhin bevorzugt mehr als 90 Gew.-% an 2,4,4-Trimethylpent-1-en, besonders bevorzugt mehr als 95 Gew.-% an 2,4,4-Trimethylpent-1-en enthält, und einen Reststrom, der gegenüber dem eingesetzten Di-isobutenstrom an 2,4,4-Trimethylpent-1-en abgereichert ist, aufgetrennt.

Der in Schritt a eingesetzte Di-isobutenstrom enthält 2,4,4-Trimethylpent-2-en und 2,4,4-Trimethylpent-1-en. Derartige Ströme können Di-isobutenströme sein, die mittels Dimerisierung aus Isobuten oder Isobutenhaltigen Kohlenwasserstoffgemischen hergestellt werden können, beispielsweise so wie es in EP 1 360 160 B1 offenbart wird. Weiterhin können die hier einzusetzenden Ströme als nicht reagierte Restströme bei Carbonylierungsverfahren anfallen, beispielsweise bei der Methoxycarbonylierung oder bei der Hydroformylierung. Es ist bereits erwähnt worden, dass der Einsatzstrom zur Isomerisierung und/oder zur Destillation zugeführt werden kann. Die Bezeichnung der Schritte a. und b. im vorliegenden Anspruch stellt somit keine Priorisierung oder Chronologie dar. Es versteht sich, dass beide Schritte durchgeführt werden müssen und dass die einzelnen Schritte aus dem jeweils anderen Schritt gespeist werden. Welcher Schritt also als zuerst ausgeführt angesehen wird, ist daher nebensächlich.

Die Destillation in Schritt a des erfindungsgemäßen Verfahrens wird in mindestens einer Destillationskolonne durchgeführt. Destillationskolonnen sind dem Fachmann grundsätzlich bekannt. Die Destillationseinheit der vorliegenden Erfindung umfasst vorzugsweise mindestens eine Destillationskolonne, besonders bevorzugt mindestens zwei Destillationskolonnen. Die nachfolgende Beschreibung von Merkmalen der Destillationskolonne gilt immer auch für den Fall, dass mehr als eine Destillationskolonne in der Destillationseinheit vorliegt.

Die mindestens eine Destillationskolonne weist vorzugsweise Einbauten auf, um die Trennaufgabe zu bewältigen. Entsprechende Einbauten sind dem Fachmann geläufig. Besonders geeignet sind hier regellose Packungen oder Strukturpackungen, wie sie dem Fachmann unter Handelsnamen wie MellaPak^{®}, MellapakPlus^{®}, Flexipac^{®} etc. bekannt sind. In einer bevorzugten Ausführungsform der vorliegenden Erfindung umfasst die mindestens eine Destillationskolonne mindestens 50 theoretische Trennstufen, vorzugsweise mindestens 70 theoretische Trennstufen, besonders bevorzugt mindestens 90 theoretische Trennstufen. Liegen zwei oder mehr Destillationskolonnen vor, können die Destillationskolonnen eine identische oder eine unterschiedliche Anzahl an theoretischen Trennstufen aufweisen.

Die Betriebsparameter der mindestens einen Destillationskolonne orientieren sich an der Trennaufgabe und der Gestaltung der Destillationskolonne. Im vorliegenden Verfahren ist es bevorzugt, dass die mindestens eine Destillationskolonne bei Unterdruck, besonders bevorzugt bei einem Druck von 0,2 bis 0,9 bar betrieben wird. Unterdruck liegt im Rahmen der vorliegenden Erfindung immer dann vor, wenn unterhalb des Umgebungsdrucks von ca. 1 bar, also dem an dem jeweiligen Standort vorliegenden Atmosphärendruck gearbeitet wird. Liegen zwei oder mehr Destillationskolonnen vor, können die Destillationskolonnen beim gleichen oder bei einem unterschiedlichen Druck betrieben werden.

Es ist weiterhin bevorzugt, dass die Temperatur im Sumpf der mindestens einen Destillationskolonne im Bereich von 50 bis 100 °C liegt. Liegen zwei oder mehr Destillationskolonnen vor, können die Destillationskolonnen bei der gleichen oder bei unterschiedlicher Temperatur betrieben werden.

Ein weiterer Parameter bei der Auslegung von Destillationskolonnen ist das Rücklaufverhältnis. Das Rücklaufverhältnis meint das Verhältnis Rücklauf (Rezyklat) zum Destillat (abgezogenes Kondensat, hier also der Kopfstrom K). Der Rücklauf ist also ein auskondensierter Teil des Kopfstroms, der in die Destillationskolonne zurückgeführt wird. Es ist erfindungsgemäß bevorzugt, dass das Rücklaufverhältnis der mindestens einen Destillationskolonne bei der Destillation in Schritt a. im Bereich von 5 bis 15 beträgt.

Durch die erfindungsgemäße Destillation in Schritt a fällt an der mindestens einen Destillationskolonne ein Kopfstrom an, der vorzugsweise mindestens 85 Gew.-% an 2,4,4-Trimethylpent-1-en, weiterhin bevorzugt mindestens 90 Gew.-%an 2,4,4-Trimethylpent-1-en, besonders bevorzugt mindestens 95 Gew.-% an 2,4,4-Trimethylpent-1-en enthält. Weiterhin fällt bei der Destillation ein Reststrom an, der gegenüber dem eingesetzten Di-isobutenstrom an 2,4,4-Trimethylpent-1-en abgereichert ist.

Der zusätzlich bei der Destillation in Schritt a anfallende Reststrom kann als Sumpfstrom oder als Seitenstrom an der mindestens einen Destillationskolonne abgenommen werden. Sind zwei Destillationskolonnen vorhanden, wird der Reststrom, unabhängig davon, ob als Seitenstrom oder Sumpfstrom abgenommen, in der letzten Destillationskolonne anfallen. Wird der Reststrom als Seitenstrom abgenommen kann er grundsätzlich auf gleicher Höhe oder unterhalb des Einlasses für den eingesetzten Di-isonbutenstrom angeordnet sein. Dabei versteht sich, dass Zulauf und Ablauf ausreichend beanstandet voneinander angeordnet sein müssen. Der Reststrom, unabhängig davon, ob als Seitenstrom oder Sumpfstrom abgenommen, enthält vorzugsweise 80 bis 92 Gew.-% 2,4,4-Trimethylpent-2-en.

Wird der Reststrom bei der Destillation in Schritt a als Sumpfstrom der mindestens einen Destillationskolonne abgenommen, wird vorzugsweise ein Purgestrom aus dem Reststrom abgezogen, der während der Isomerisierung entstandene Hochsieder, z. B. Dimere oder Oligomerisate von 2,4,4-Trimethylpent-2-en und/oder 2,4,4-Trimethylpent-1-en, enthält. Die abgetrennten Hochsieder können zur Energieerzeugung verbrannt oder einer Hydrierung unterworfen werden, um daraus werthaltige Alkane herzustellen.

Wird der Reststrom bei der Destillation in Schritt a als Seitenstrom der mindestens einen Destillationskolonne abgenommen, wird vorzugsweise im Sumpf der mindestens einen Destillationskolonne ein Strom anfallen, der die während der Isomerisierung entstandenen Hochsieder, z. B. Dimere oder Oligomerisate von 2,4,4-Trimethylpent-2-en und/oder 2,4,4-Trimethylpent-1-en, enthält. Die abgetrennten Hochsieder können zur Energieerzeugung verbrannt oder einer Hydrierung unterworfen werden, um daraus werthaltige Alkane herzustellen.

Destillationskolonnen werden bekanntermaßen im Sumpf beheizt, um die Trennaufgabe bewältigen zu können. Möglich ist hier eine Energiezufuhr über Heizdampf, der oft an chemischen Produktionsstandorten verfügbar ist. Dazu wird ein Teil des Sumpfes über einen Wärmetauscher (Sumpfverdampfer) geführt, dort erhitzt und dann zurück in den Sumpf der Destillationskolonne geführt. Um den Energiebedarf und/oder um die COz-Emissionen zu verringern, kann eine Wärmeintegration bei der Destillation in Schritt a. vorgesehen werden. Wärmeintegration bedeutet, dass Energie, die innerhalb des Verfahrens entsteht oder vorhanden ist, an anderer Stelle eingesetzt wird. Im vorliegenden Fall eignet sich vor allem die (freiwerdende) Kondensationsenergie, die am Kopf mindestens einer Destillationskolonne der Destillationseinheit anfällt. In einer bevorzugten Ausführungsform der vorliegenden Erfindung wird in Schritt a. also eine Wärmeintegration dergestalt durchgeführt, dass zumindest ein Teil der Kondensationsenergie am Kopf zum Erhitzen des Sumpfs genutzt wird.

Eine andere Möglichkeit zur Wärmeintegration besteht in der sogenannten Brüdenverdichtung, bei der zumindest ein Teil des Brüdens (Kopfstroms) verdichtet, also auf ein höheres Druckniveau gebracht, und optional erhitzt wird. Der so verdichtete und optional erhitzte Brüden wird zum Wärmetauscher (Sumpfverdampfer) geführt, um damit den Sumpf zu erhitzen. Hierzu wird die Kondensationswärme des Brüden genutzt.

Eine weitere Möglichkeit besteht in dem Einsatz einer Wärmepumpe. Wärmepumpen werden mit einem Arbeitsmedium, z. B. n-Butan oder Wasser betrieben. Die Kondensationsenergie wird hier also zunächst in einem Wärmetauscher auf ein Arbeitsmedium übertragen und von diesem in einem weiteren geeigneten Wärmetauscher auf den Sumpf in der Destillation. Das Arbeitsmedium wird dabei in aller Regel über einen Verdichter zum Wärmetauscher im Sumpf geführt. Es ist grundsätzlich auch möglich zweistufige oder mehrstufige Wärmepumpen einzusetzen, bei denen mehr als eine Kompressorstufe vorliegt. Bei einer zweistufigen Wärmepumpe liegt nicht nur ein Arbeitsmedium, sondern zwei Arbeitsmedien vor, wobei zwischen dem ersten und dem zweiten Arbeitsmedium ebenfalls ein Energieaustausch in einem Wärmetauscher stattfindet. Bei mehrstufigen Ausgestaltungen sind entsprechend mehr Arbeitsmedien vorhanden.

Der aus der Destillation in Schritt a erhaltene Kopfstrom wird dem nachfolgenden Schritt b der Alkoxycarbonylierung zugeführt und dort umgesetzt. Der ebenfalls aus der Destillation in Schritt a erhaltene Reststrom wird nach einer bevorzugten Ausführungsform zu einer Isomerisierung geführt, bei der 2,4,4-Trimethylpent-2-en unter Verwendung eines heterogenen Katalysators auf Basis eines Zeoliths oder eines Ionenaustauscherharzes zumindest teilweise 2,4,4-Trimethylpent-1-en isomerisiert wird. Die Isomerisierung kann also mit dem Reststrom durchgeführt, der gegenüber dem in Schritt a eingesetzten Di-isobutenstrom an 2,4,4-Trimethylpent-1-en abgereichert ist und aus der Destillation in Schritt a entnommen wird.

Die Durchführung einer Isomerisierung vor der Destillation in Schritt a hat den Vorteil, dass ein heterogenes Katalysatorsystems eingesetzt wird. Ein solches Katalysatorsystem muss nicht vom Isomerisierungsstrom abgetrennt werden und verbleibt im Reaktionsgefäß bzw. dem Reaktor. Mit den erfindungsgemäß bevorzugten Katalysatorsystemen auf Basis eines Zeoliths oder eines Ionenaustauscherharzes können zudem hohe Umsätze erzielt werden.

Die Isomerisierung kann grundsätzlich in jedem geeigneten Reaktor durchgeführt werden. Möglich ist, dass die Isomerisierung in einem einzigen Reaktor oder in mehreren parallel oder in Reihe geschalteten Reaktoren erfolgt. Möglich ist zudem die Durchführung in Chargen oder im kontinuierlichen Betrieb. Bevorzugt wird die Isomerisierung in einem oder mehreren kontinuierlich arbeitenden Reaktoren, die üblicherweise bei Feststoff/Flüssigkeits-Kontaktreaktionen zum Einsatz gelangen, durchgeführt. Bei der Verwendung von kontinuierlich arbeitenden Strömungsreaktoren bedient man sich meistens, jedoch nicht ausschließlich, eines Festbetts. Wenn ein Festbett-Strömungsreaktor verwendet wird, kann die Flüssigkeit aufwärts oder abwärts strömen. Meistens wird ein Abwärtsströmen der Flüssigkeit bevorzugt. Weiterhin ist es möglich, den Reaktor unter Produktrückführung oder im geraden Durchgang zu betreiben. Ein anderes Konzept als Festbettreaktoren sind beispielsweise Reaktoren, in denen der Ionenaustauscher oder Zeolith suspendiert in einer flüssigen Phase vorliegt.

Als Reaktoren können bei der Isomerisierung Rohrreaktoren oder Rohrbündelreaktoren, insbesondere solche mit inneren Rohrdurchmessern von 10 bis 60 mm, verwendet werden. Das Verhältnis von Länge zu Durchmesser der Katalysatorschüttung kann dabei variiert werden, entweder durch die geometrischen Maße des Reaktors oder durch dessen Füllgrad. Bei gleicher Kontaktmenge und Belastung (LHSV) können somit unterschiedliche Leerrohrgeschwindigkeiten erreicht und der Wärmeübergang an das Kühlmedium gezielt beeinflusst werden.

Die Kühlung der Rohre des Reaktors, sei es Rohrreaktor oder Rohrbündelreaktor, kann über ein Kühlmedium (z.B. Kühlwasser oder zur Wärmeintegration ein wärmeaufnehmendes Prozessfluid) über den Mantelraum des Reaktors oder einen Wärmeübertrager in einem externen Recycle erfolgen. Insbesondere bei Einsatz von flüssigen Heizmedien wird dabei die Mantelseite konstruktiv so ausgeführt, dass ein möglichst homogener Temperaturgradient an allen Rohren anliegt. Die hierfür notwendigen technischen Maßnahmen sind dem Fachmann bekannt und in der Literatur beschrieben (Einbau von Umlenkblechen, Disc- an Donut-Bauweise, Einspeisung / Abfuhr des Wärmeträgers an verschiedenen Stellen des Reaktors usw.). Bevorzugt werden Reaktionsmedium und Wärmeträger im Gleichstrom, besonders bevorzugt von oben nach unten durch die Reaktorrohre bzw. den Reaktormantel geführt. Eine bevorzugte Ausführungsform ist beispielsweise in DE 10 2006 040 433 A1 beschrieben.

Die Isomerisierung von 2,4,4-Trimethylpent-2-en zu 2,4,4-Trimethylpent-1-en erfolgt exotherm, verläuft also unter Freisetzung von Energie, was zu einer Erwärmung der Reaktionsmischung führt. Zur Begrenzung des Temperaturanstiegs ist eine Verdünnung des Einsatzstroms, beispielsweise durch Rückführung von Produkt möglich.

Der oder die bei der Isomerisierung eingesetzten Reaktor(en) können adiabatisch, polytrop oder praktisch isotherm betrieben werden. Praktisch isotherm bedeutet, dass die Temperatur an einer beliebigen Stelle im Reaktor maximal um 10°C höher ist als die Temperatur am Reaktoreingang. Bei adiabatischem Betrieb der Reaktoren ist es in der Regel sinnvoll, mehrere Reaktoren in Reihe zu schalten und zwischen den Reaktoren eine Kühlung vorzusehen. Reaktoren, die für einen polytropen oder praktisch isothermen Betrieb geeignet sind, sind beispielsweise die bereits erwähnten Rohrbündelreaktoren, aber auch Rührkessel- und Schlaufenreaktoren.

Das Verfahren kann bei eher milden Temperaturen durchgeführt werden. Die Isomerisierung wird vorzugsweise bei einer Temperatur von 25 bis 90 °C, vorzugsweise 30 bis 80 °C, besonders bevorzugt 35 bis 70 °C durchgeführt. Weiterhin kann die erfindungsgemäße Isomerisierung bei einem Druck gleich oder größer als der Dampfdruck des Einsatzstromgemisches und/oder des Reaktionsgemisches bei der jeweiligen Reaktionstemperatur durchgeführt werden, vorzugsweise bei einem Druck von mehr als 0 bar aber weniger als 40 bar. Weiterhin bevorzugt wird die Isomerisierung in der Flüssigphase durchgeführt. Es sollte klar sein, dass in diesem Fall Druck und Temperatur so zu wählen sind, dass der Einsatzstrom in der flüssigen Phase vorliegt bzw. vorliegen kann.

In dem oder den Reaktor(en) können bei der Isomerisierung auch verschiedene Katalysatoren auf Basis eines Zeoliths oder eines Ionenaustauscherharzes eingesetzt werden. So kann beispielsweise ein Gemisch von lonenaustauscherharzen unterschiedlicher Reaktivität eingesetzt werden. Ebenso ist es möglich, dass ein Reaktor Katalysatoren mit unterschiedlicher Aktivität enthält, die beispielsweise in Schichten angeordnet sind. Wird mehr als ein Reaktor verwendet, können die einzelnen Reaktoren mit dem gleichen oder unterschiedlichen Katalysator(en) auf Basis eines Zeoliths oder eines Ionenaustauscherharzes gefüllt sein.

Als heterogener Katalysator kann bei der Isomerisierung ein Katalysator auf Basis eines Zeoliths oder eines Ionenaustauscherharzes eingesetzt werden. Entsprechende Zeolithe und lonenaustauscherharze sind zahlreich im Handel verfügbar. Es hat sich gezeigt, dass der Katalysator auf Basis eines Zeoliths vorzugsweise ein Si : Al Verhältnis im Bereich von 40 : 1 bis 200 : 1 aufweist. Weiterhin hat sich gezeigt, dass als Katalysator auf Basis eines Ionenaustauscherharzes vorzugsweise der Styrol-Divinylbenzol-Typ als H-Form und in teilneutraliserter Form gut geeignet ist.

Ist der Katalysator ein Zeolith, haben sich einige Zeolithe als besonders vorteilhaft herausgestellt, beispielsweise beta- und gamma-Zeolithe. Der Zeolith wird daher vorzugsweise aus der Gruppe bestehend aus Z-beta-H-25, Z-beta-H-38, Z-beta-H-360, Z-Mor-H-20, Z-Y-H-60, Z-Y-H-80, Z-beta-H, Z-CFG-1, Z-beta-ammonium-38, Z-CBV 760 CY (1.6), Z-CBV 780 CY (1.6), CP 814E CY (1.6), CBV 500 CY (1.6), H-CZB-150 und Mischungen davon ausgewählt.

Als lonenaustauscherharz können beispielsweise lonenaustauscherharze eingesetzt werden, solche, die durch die Sulfonierung von Phenol/Aldehyd-Kondensaten oder durch die Sulfonierung von Copolymeren von aromatischen Vinylverbindungen hergestellt werden. Beispiele für aromatische Vinylverbindungen zur Herstellung der Copolymere sind: Styrol, Vinyltoluol, Vinylnaphthalin, Vinylethylbenzol, Methylstyrol, Vinylchlorbenzol, Vinylxylol und Divinylbenzol. Besonders bevorzugt werden lonenaustauscherharze für die Isomerisierung eingesetzt, die durch die Sulfonierung von Copolymerenn, die durch Umsetzung von Styrol mit Divinylbenzol entstehen, hergestellt werden. Die lonenaustauscherharze können gelförmig, makroporös oder schwammförmig hergestellt werden. Die Eigenschaften dieser Harze, insbesondere spezifische Oberfläche, Porosität, Stabilität, Quellung oder Schrumpfung und Austauschkapazität, können bekanntermaßen durch den Herstellprozess variiert werden.

Ionenaustauscherharze des bevorzugten Styrol-Divinylbenzol-Typs werden u. a. unter folgenden Handelsnamen verkauft: CT 151 und CT275 der Firma Purolite, Amberlyst^{®} 15, Amberlyst^{®} 35, Amberlite^{®} IR-120, Amberlite^{®} 200 der Firma Rohm&Haas, Dowex M-31 der Firma DOW, LEWATIT^{®} K 2621, LEWATIT^{®} K 2431der Firma Lanxess.

Das Porenvolumen der als Katalysatoren einsetzbaren lonenaustauscherharze, insbesondere des bevorzugten Styrol-Divinylbenzol-Typs, beträgt vorzugsweise 0,3 bis 0,9 ml/g, besonders bevorzugt 0,5 bis 0,9 ml/g. Das Porenvolumen kann beispielsweise mittels adsorptiver Techniken ermittelt werden. Die Korngröße der lonenaustauscherharze beträgt bevorzugt van 0,3 mm bis 1,5 mm, besonders bevorzugt 0,5 mm bis 1,0 mm. Die Korngrößenverteilung kann enger oder weiter gewählt werden. So können beispielsweise lonenaustauscherharze mit sehr einheitlicher Korngröße (monodisperse Harze) eingesetzt werden.

Die als Katalysatoren bei der Isomerisierung einsetzbaren lonenaustauscherharze können als teilneutralisierte lonenaustauscherharze vorliegen. Dazu kann das lonenaustauscherharz mit Säuren oder Basen behandelt werden, wie es in der EP 1 360 160 B1 beschrieben wird.

Aus der optionalen Isomerisierung wird ein Isomerisierungsstrom erhalten, bei dem der Anteil des 2,4,4-Trimethylpent-2-ens geringer und der Anteil des 2,4,4-Trimethylpent-1-ens im Isomerisierungsstrom höher ist als im eingesetzten Reststrom. Der Isomerisierungsstrom kann zumindest teilweise zur Destillation in Schritt a geführt werden, vorzugsweise wird der Isomerisierungsstrom vollständig zu Destillation in Schritt a geführt.

Das Verfahren kann weiterhin das Zuführen eines Einsatzstroms umfassen, mit dem das Verfahren gespeist wird. Der Einsatzstrom, mit dem das Verfahren gespeist wird und der 2,4,4-Trimethylpent-2-en und 2,4,4-Trimethylpent-1-en enthält, kann zusätzlich zum Isomerisierungsstrom zur Destillationseinheit in Schritt a und/oder zusätzlich zum Reststrom zur Isomerisierung geleitet werden.

Dabei ist die flexible Zuführung des Einsatzstroms vorteilhaft. Dadurch kann das Verfahren nämlich an die jeweiligen Gegebenheiten angepasst werden, beispielweise in Abhängigkeit des eingesetzten Einsatzstroms. Liegen die Anteile von 2,4,4-Trimethylpent-2-en und 2,4,4-Trimethylpent-1-en im Einsatzstrom nah an der Gleichgewichtsverteilung, kann der Strom direkt zur Destillation geführt werden. Bei von der Gleichgewichtsverteilung abweichenden Anteilen von 2,4,4-Trimethylpent-2-en und 2,4,4-Trimethylpent-1-en im Einsatzstrom könnte der Strom zunächst zur Isomerisierung geführt werden. Der Einbau in bestehende Produktionsanlagen ist somit in Abhängigkeit von der Zusammensetzung des Einsatzstroms auf einfache Weise möglich.

Der aus der Destillation in Schritt a erhaltene Kopfstrom wird, wie erwähnt, dem nachfolgenden Schritt b der Alkoxycarbonylierung zugeführt und dort umgesetzt.

Die Di-isobutene, d. h. 2,4,4-Trimethylpent-2-en und 2,4,4-Trimethylpent-1-en, werden in Schritt b mit Kohlenmonoxid (CO) und einem Alkohol zu einem Ester umgesetzt. Die Anzahl der Kohlenstoffatome im Ester erhöht sich dabei im Vergleich mit dem eingesetzten Di-isobuten um 1 Kohlenstoffatom aus dem Kohlenmonoxid sowie um die Anzahl der Kohlenstoffatome des eingesetzten Alkohols. Aus den Di-isobutenen (8 Kohlenstoffatome) entsteht demnach ein Ester mit 9 Kohlenstoffatomen im Säureteil des Esters plus den Kohlenstoffatomen des Alkohols im Alkoholteil des Esters.

Das Kohlenmonoxid kann in Schritt b direkt als Einsatzgemisch oder durch die Zugabe eines Kohlenmonoxid-haltigen Gases, ausgewählt aus Synthesegas, Wassergas, Generatorgas und anderen Kohlenmonoxid-haltigen Gasen bereitgestellt werden. Möglich ist es auch, das Kohlenmonoxid dadurch bereitzustellen, dass das Kohlenmonoxid-haltige Gas vorher in einer für den Fachmann bekannten Weise in seine Komponenten aufgetrennt wird und das Kohlenmonoxid zur Reaktionszone geleitet wird. Das Kohlenmonoxid kann dabei weiterhin einen gewissen Anteil an Wasserstoff oder anderen Gasen enthalten, weil eine vollständige Auftrennung kaum zu realisieren ist.

Der bei der Umsetzung in Schritt b eingesetzte Alkohol ist vorzugsweise ein einwertiger Alkohol mit 1 bis 4 Kohlenstoffatomen, insbesondere Methanol, Ethanol, Propanol oder Butanol. Dadurch werden dann die Ester mit dem eingesetzten Alkohol entsprechender Kettenlänge erhalten. Bei Einsatz von Methanol entsteht beispielsweise der Methylester.

Der in Schritt b eingesetzte Alkohol, vorzugsweise der einwertige Alkohol mit 1 bis 4 Kohlenstoffatomen, insbesondere Methanol, Ethanol, Propanol oder Butanol, wird in einem Molverhältnis zur Gesamtmenge aller Di-isobutene, Alkohol: Di-isobutene in einem Bereich von 10 : 1 bis 1 : 1, vorzugsweise 8 : 1 bis 1,5 : 1, besonders bevorzugt 7 : 1 bis 2 : 1 eingesetzt. Der Alkohol wird somit - bezogen auf die eingesetzten Di-isobutene - mindestens in identischer molarer Menge, vorzugsweise aber im molaren Überschuss hinzugefügt.

Die erfindungsgemäße Umsetzung in Schritt b wird in Gegenwart eines homogenen Katalysatorsystems durchgeführt, welches zumindest ein Metall aus der Gruppe 8 bis 10 des Periodensystems der Elemente (PSE) oder eine Verbindung davon, einen phosphorhaltigen Liganden und eine Säure als Co-Katalysator umfasst. Der Gehalt des Metalls der Gruppe 8 bis 10 des Periodensystems der Elemente, insbesondere des Palladiums, beträgt in der der Reaktionslösung bei der Alkoxycarbonylierung in Schritt b vorzugsweise 100 bis 500 ppm, weiterhin bevorzugt 150 bis 450 ppm, besonders bevorzugt 180 bis 350 ppm.

Das Metall aus der Gruppe 8 bis 10 des PSE ist vorzugsweise Palladium. Das Palladium wird bevorzugt in Form einer Vorläuferverbindung als Palladiumverbindung eingesetzt, die durch den phosphorhaltigen Liganden koordiniert wird. Beispiele für Palladiumverbindungen, die als Vorläuferverbindungen eingesetzt werden können, sind Palladiumchlorid [PdCl₂], Palladium(II)-Acetylacetonat [Pd(acac)₂], Palladium(II)-Acetat [Pd(OAc)₂], Dichloro-(1,5-cyclooctadien)palladium(II) [Pd(cod)₂Cl2], Bis(dibenzylideneaceton)palladium(0) [Pd(dba)₂], Tris(dibenzylideneaceton)dipalladium(0) [Pd₂(dba)₃] Bis(acetonitril)-dichloropalladium(II) [Pd(CH₃CN)₂Cl₂], Palladium(cinnamyl)-dichlorid [Pd(cinnamyl)Cl₂]. Vorzugsweise kommen die Verbindungen [Pd(acac)₂] oder [Pd(OAc)₂] zum Einsatz. Die Metallkonzentration von Palladium in Schritt b beträgt vorzugsweise zwischen 0,01 und 0,6 Mol-%, bevorzugt zwischen 0,03 und 0,3 Mol-%, besonders bevorzugt zwischen 0,04 und 0,2 Mol-% bezogen auf die Stoffmenge des eingesetzten Kohlenwasserstoffs.

Geeignete phosphorhaltige Liganden des erfindungsgemäßen Katalysatorsystems weisen vorzugsweise eine Bidentat-Struktur auf. Bevorzugte phosphorhaltige Liganden für das erfindungsgemäße Katalysatorsystem sind benzolbasierte Diphosphinverbindungen, wie sie beispielsweise in der EP 3 121 184 A2 offenbart werden. Die Liganden können in einer Vorreaktion mit dem Palladium kombiniert werden, sodass der Palladium-Ligand-Komplex zur Reaktionszone geführt wird, oder in situ zur Reaktion gegeben und dort mit dem Palladium kombiniert werden. Das Molverhältnis von Ligand : Metall kann für die beschriebene Umsetzung in Schritt b 1 : 1 bis 10 : 1, vorzugsweise 2 : 1 bis 6 : 1, besonders bevorzugt 3 : 1 bis 5 : 1 betragen.

Das homogene Katalysatorsystem umfasst weiterhin eine Säure, wobei es sich insbesondere um eine Brönsted- oder eine Lewis-Säure handelt. Als Lewis-Säure werden vorzugsweise Aluminiumtriflat, Aluminiumchlorid, Aluminiumhydrid, Trimethylaluminium, Tris(pentafluorophenyl)boran, Bortrifluorid, Bortrichlorid oder Mischungen daraus eingesetzt werden. Von den genannten Lewis-Säuren wird bevorzugt Aluminiumtriflat eingesetzt. Die Lewis-Säure wird vorzugsweise in einem Molverhältnis Lewis-Säure : Ligand von 1 : 1 bis 20 : 1, vorzugsweise 2 : 1 bis 15 : 1, besonders bevorzugt 5 : 1 bis 10 : 1 hinzugegeben.

Geeignete Brönsted-Säuren haben vorzugsweise eine Säurestärke von pKs ≤ 5, besonders bevorzugt eine Säurestärke von pKs ≤ 3. Die angegebene Säurestärke pKs bezieht sich auf den bei Normalbedingungen (25°C, 1,01325 bar) bestimmten pKs-Wert. Bei einer mehrprotonigen Säure bezieht sich die Säurestärke pKs im Rahmen dieser Erfindung auf den pKs-Wert des ersten Protolyseschrittes. Die Brönsted-Säure wird vorzugsweise in einem Molverhältnis Brönsted-Säure : Ligand von 1 : 1 bis 15 : 1, vorzugsweise 2 : 1 bis 10 : 1, besonders bevorzugt 3 : 1 bis 5 : 1 hinzugegeben.

Als Brönsted-Säuren können insbesondere Perchlorsäure, Schwefelsäure, Phosphorsäure, Methylphosphonsäure oder Sulfonsäuren eingesetzt werden. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure. Vorzugsweise handelt es sich bei der Säure um Schwefelsäure.

Die Alkoxycarbonylierung in Schritt b wird vorzugsweise bei einer Temperatur im Bereich von 60 bis 120 °C, weiterhin bevorzugt im Bereich von 65 bis 110 °C, besonders bevorzugt im Bereich von 70 bis 100 °C durchgeführt. Die Alkoxycarbonylierung in Schritt b wird weiterhin bevorzugt bei einem Kohlenmonoxiddruck von 10 bis 35 bar, vorzugsweise 12,5 bis 30 bar, besonders bevorzugt 15 bis 25 bar durchgeführt.

Die Alkoxycarbonylierung in Schritt b findet in einer geeigneten Reaktionszone statt. Die Reaktionszone für die Umsetzung umfasst mindestens einen Reaktor, kann aber auch aus zwei oder mehr Reaktoren bestehen, die parallel oder in Reihe geschaltet angeordnet sind. Der mindestens eine Reaktor kann insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich voneinander sein.

Durch die oben beschriebene Alkoxycarbonylierung in Schritt b wird ein flüssiges Produktgemisch erhalten, das zumindest den durch die Alkoxycarbonylierung gebildeten Ester, das homogene Katalysatorsystem, sie nicht umgesetzte Di-isobutene 2,4,4-Trimethylpent-2-en und 2,4,4-Trimethylpent-1-en und nicht umgesetzten Alkohol umfasst. Zusätzlich kann das flüssige Produktgemisch leichtsiedende Nebenprodukte wie Dimethylether oder Ameisensäure und/oder hochsiedende Komponenten wie Ligandenabbauprodukte enthalten.

Das so erhaltene Produktgemisch wird dem nachfolgenden Schritt c zugeführt, um das homogene Katalysatorsystems aus dem flüssigen Produktgemisch abzutrennen. Vor dem Zuführen des Produktgemisch können bereits leichtsiedende Komponenten beispielsweise ein Teil des nicht umgesetzten Alkohols und/oder der leichtsiedenden Nebenprodukte abgetrennt werden. Der Alkohol kann zu Schritt b, also der Alkoxycarbonylierung zurückgeführt werden. Die leichtsiedenden Nebenprodukte können aus dem Verfahren im Sinne eines Purge ausgeschleust werden, damit sie sich nicht im Verfahren anreichern. Aus der Abtrennung des homogenen Katalysatorsystems wird ein Rohproduktgemisch erhalten, welches zumindest die durch die Alkoxycarbonylierung gebildeten Ester, nicht umgesetzte Di-isobutene und zumindest einen Teil der nicht umgesetzten Alkohole umfasst.

Die Abtrennung des homogenen Katalysatorsystems unter Erhalt des Rohproduktgemisches in Schritt c kann mithilfe verschiedener Trennverfahren erfolgen, beispielsweise mittels thermischer Trennung und/oder durch Membrantrennung. Entsprechende Verfahren sind dem Fachmann geläufig. Vorzugsweise erfolgt die Abtrennung des homogenen Katalysatorsystems in Schritt c im Rahmen der vorliegenden Erfindung vorzugsweise mittels Membrantrennung. Bei einer Membrantrennung fallen bekanntermaßen ein Retentat und ein Permeat an. Im Retentat wird sich das homogene Katalysatorsystem anreichern. Das Permeat stellt dann das bereits erwähnte Rohproduktgemisch dar und wird zum nachfolgenden Schritt d, der destillativen Aufarbeitung, geleitet.

Es ist erfindungsgemäße bevorzugt, dass das Retentat zur Alkoxycarbonylierung in Schritt b bzw. zur Reaktionszone, wo die Alkoxycarbonylierung durchgeführt wird, zurückgeführt wird. So kann das Katalysatorsystem wiederverwendet werden. Bei der bevorzugt kontinuierlichen Durchführung des beanspruchten Verfahrens entsteht so ein Katalysatorkreislauf, wo wenn überhaupt nur geringfügige prozessbedinge Katalysatorverluste ausgeglichen werden müssen. Sofern der Di-isobutenstrom, der Alkohol und das homogene Katalysatorsystem gemäß der bevorzugten Ausführungsform vor der Alkoxycarbonylierung in Schritt b insbesondere in einem geeigneten Mischbehälter vermischt werden wird, wird das Retentat zum Mischbehälter geführt. Bei der Rückführung des Retentats kann weiterhin ein Purgestrom, welcher inerte Alkane, leichtsiedende Nebenprodukte (bspw. Ether), mögliche Abbauprodukte des Katalysatorsystems oder andere Verunreinigungen wie beispielsweise Spuren von Wasser oder Stickstoff enthalten kann, entnommen werden, um eine Akkumulation in der oder den Reaktionszonen zu vermeiden.

Bei der Membrantrennung kann jedes geeignete Membranmaterial eingesetzt werden. Vorzugsweise wird bei der Membrantrennung in Schritt c des erfindungsgemäßen Verfahrens ein OSN-Membranmaterial (OSN = Organic Solvent Nanofiltration) eingesetzt. Ein solches Membranmaterial besteht vorzugsweise zumindest aus einer trennaktiven Schicht (auch: aktiven Trennschicht) und einer Unterstruktur, worauf sich die trennaktive Schicht befindet. Bevorzugt besteht das erfindungsgemäße Membranmaterial zumindest aus einer trennaktiven Schicht und einer Unterstruktur.

Das Membranmaterial, aus zumindest trennaktiver Schicht und einer Unterstruktur, sollte säurestabil sein, damit das Membranmaterial nicht durch die im flüssigen Produktgemisch befindliche Säure beschädigt wird. Der Begriff "säurestabil" meint im Rahmen der vorliegenden Erfindung, dass das Membranmaterial mindestens 300 h in Anwesenheit der Säure des Katalysatorsystems stabil ist und die Trennleistung erhalten bleibt.

Die Unterstruktur weist vorzugsweise eine poröse Struktur auf, die für das durch die trennaktive Schicht gelangte Permeat durchlässig ist. Die Unterstruktur hat eine stabilisierende Funktion und dient als Träger für die trennaktiven Schicht. Die Unterstruktur kann grundsätzlich aus jedem geeigneten porösen Material bestehen. Entsprechende Materialien sind dem Fachmann geläufig. Voraussetzung ist jedoch, dass das Material säure- und basenstabil ist. Die Unterstruktur kann auch aus dem gleichen Material bestehen wie die trennaktive Schicht. Bevorzugte Materialien für die Unterstruktur sind Kunststoffe wie Polypropylen oder einem PAEK-Polymer PAEK steht für Polyaryletherketon. Das PAEK-Polymer ist insbesondere PEEK (Polyetheretherketon), besonders bevorzugt ein PEEK-Polymer mit einem Sulfonierungsgrad von weniger als 20%, besonders bevorzugt mit einem Sulfonierungsgrad von weniger als 10%

Die erfindungsgemäße trennaktive Schicht besteht vorzugsweise aus einem PAEK-Polymer (Polyaryletherketon). PAEK zeichnet sich dadurch aus, dass innerhalb der Widerholungseinheit Arylgruppen abwechselnd über eine Etherfunktionalität und eine Ketonfunktionalität verknüpft sind. Eine erfindungsgemäß bevorzugte trennaktive Schicht besteht aus PEEK (Polyetheretherketon). Als trennaktive Schicht können insbesondere bevorzugt PEEK-Polymere mit einem Sulfonierungsgrad von weniger als 20%, besonders bevorzugt mit einem Sulfonierungsgrad von weniger als 10% eingesetzt werden. Die entsprechenden PEEK-Polymere und deren Herstellung sind in der WO 2015/110843 A1 beschrieben.

Die Membrantrennung in Schritt cwird vorzugsweise bei einer Temperatur im Bereich von 25 bis 100°C, weiterhin bevorzugt im Bereich von 30 bis 80°C und besonders bevorzugt im Bereich von 40 bis 70°C durchgeführt. Um das Produktgemisch auf die bei der Membrantrennung bevorzugte vorherrschende Temperatur zu bringen, kann das Produktgemisch gekühlt werden. Neben einer aktiven Kühlung unter Verwendung eines Kühlmediums, kann die Kühlung auch über einen Wärmetauscher erreicht werden, wodurch ein anderer Strom innerhalb des erfindungsgemäßen Verfahrens erhitzt wird.

Der Transmembrandruck (TMP) liegt bei der Membrantrennung in Schritt c vorzugsweise im Bereich von 10 bis 60 bar, weiterhin bevorzugt im Bereich von 15 bis 55 bar, besonders bevorzugt im Bereich von 20 bis 50 bar. Der permeatseitige Druck kann dabei oberhalb des atmosphärischen Drucks liegen und vorzugsweise bis 15 bar, vorzugsweise 3 bis 7 bar betragen. Aus der Differenz von TMP und permeatseitigem Druck ergibt sich der retentatseitige Druck. In einer bevorzugten Ausführungsform sollte bei den Druckverhältnissen und insbesondere beim permeatseitigen Druck darauf geachtet werden, dass der Druck in Abhängigkeit von dem eingesetzten Kohlenwasserstoff, dem eingesetzten Alkohol und der vorhandenen Temperatur so eingestellt wird, dass eine Verdampfung nach dem Durchtritt durch die Membran vermieden wird. Ein Verdampfen könnte zu einer instabilen Fahrweise führen.

Im anschließenden Schritt d erfolgt die destillative Aufarbeitung des Rohproduktgemisches bzw. des Permeats aus der Membrantrennung in mindestens einer Destillationskolonne zur Abtrennung der nicht umgesetzten Alkohole und der nicht umgesetzten Di-isobutene. Dabei wird ein Esterprodukt erhalten, welches die gebildeten Ester enthält. Das Esterprodukt enthält die aus dem Di-isobuten gebildeten Ester, also die 3,5,5-Trimethylhexansäurealkylester, wobei der Alkylrest je nach eingesetztem Alkohol 1 bis 4 Kohlenstoffatome umfasst.

Bei der destillativen Aufarbeitung des Rohproduktgemisches bzw. des Permeats in Schritt d fallen der nicht umgesetzte Alkohol und die nicht umgesetzten Di-isobutene am Kopf der mindestens einen Destillationskolonne an. Das Esterprodukt fällt folglich im Sumpf der mindestens einen Destillationskolonne an. Der Kopfstrom, der den in der mindestens einen Destillationskolonne abgetrennten nicht umgesetzten Alkohol und die nicht umgesetzten Di-isobutene enthält, wird zur Alkoxycarbonylierung in Schritt b zurückgeführt. Ist eine Vermischung der eingesetzten Komponenten vor der Alkoxycarbonylierung vorhanden, wird der Kopfstrom natürlich zur Vermischung geführt. Dadurch ist ein kontinuierlicher Betrieb des erfindungsgemäßen Verfahrens unter höchstmöglicher Ausbeute möglich. Aus dem zurückgeführten Kopfstrom kann ein Purge abgezogen werden, um leichtsiedende Nebenprodukte aus dem Verfahren auszuschleusen.

Die destillative Aufarbeitung zur Abtrennung der nicht umgesetzten Alkohole und der nicht umgesetzten Di-isobutene in Schritt d kann in einer Destillationskolonne erfolgen. Denkbar wäre, dass die destillative Aufarbeitung zur Abtrennung der nicht umgesetzten Alkohole und der nicht umgesetzten Di-isobutene in Schritt d in mehreren Destillationskolonnen erfolgt. Dies würde aber einen deutlichen höheren apparativen Aufwand bedeuten. Bevorzugt ist deshalb, dass die destillative Aufarbeitung in Schritt d in einer einzigen Destillationskolonne erfolgt.

Der Druck in der Destillationskolonne bei der destillativen Aufarbeitung in Schritt d liegt vorzugsweise im Bereich von 0,3 bis 2 bar, weiterhin bevorzugt im Bereich von 0,4 bis 1 bar, besonders bevorzugt im Bereich 0,5 bis 0,7 bar. Die Temperatur im Sumpf der Destillationskolonne bei der destillativen Aufarbeitung in Schritt d liegt vorzugsweise im Bereich von 80 °C bis 160 °C. Die Temperatur am Kopf der Destillationskolonne bei der destillativen Aufarbeitung in Schritt d liegt vorzugsweise im Bereich von 30 bis 80 °C. Weiterhin ist es bevorzugt, dass das Rücklaufverhältnis in der Destillationskolonne zwischen 1 und 2 beträgt. Die Destillationskolonne für die Abtrennung in Schritt d umfasst vorzugsweise 10 bis 30 theoretische Stufen. Die Destillationskolonne kann dabei Hochleistungsstrukturpackungen enthalten. Entsprechende Hochleistungsstrukturpackungen sind dem Fachmann bekannt.

Der in Schritt b hergestellte und in Schritt d abgetrennte und ggf. aufgereinigte Ester wird dann in Schritt e einer Hydrierung unterworfen. Durch den dabei eingesetzten Wasserstoff wird die Estergruppe gespalten, wodurch der Zielalkohol entsteht und der bei der Esterbildung in Schritt b angebundenen Alkohol zurückgewonnen werden kann. Es entsteht demnach bei der Hydrierung ein Alkoholgemisch, welches zumindest den Zielalkohol, den abgespaltenen Alkohol und nicht umgesetzte Ester umfasst.

Der bei der Hydrierung in Schritt e zurückgewonnene Alkohol kann in einem nachfolgenden Prozessschritt aus dem entstandenen Alkoholgemisch abgetrennt und in die erste Reaktionszone zurückgeführt werden.

Die Hydrierung ist eine an sich bekannte Reaktion. Die typischen Hydrierbedingungen sind dem Fachmann bekannt und beispielsweise in der EP 1 042 260 A1 offenbart. Die erfindungsgemäße Hydrierung in Schritt e wird vorzugsweise bei einem Druck von 10 bis 300 bar, weiterhin bevorzugt bei einem Druck von 100 bis 280 bar und besonders bevorzugt bei einem Druck von 150 bis 270 bar durchgeführt. Die Hydriertemperatur liegt vorzugsweise zwischen 100 °C und 300 °C, vorzugsweise zwischen 150 °C und 280 °C und besonders bevorzugt zwischen 180 °C und 220 °C. Bei der Hydrierung kann außerdem ein Abgas entnommen werden um leichtsiedende Komponenten, wie Verunreinigungen wie Stickstoff oder leichtsiedende Nebenprodukte zu entfernen.

Die Hydrierung in Schritt e findet in Anwesenheit eines heterogenen Katalysatorsystems statt. Typische Katalysatorsysteme sind beispielsweise aus der EP 1 338 557 A1 bekannt. Das heterogene Katalysatorsystem umfasst vorzugsweise ein Metall aus der Gruppe, bestehend aus Kupfer, Rhodium, Ruthenium, Rhenium oder Verbindungen dieser Metalle. Weiterhin sind auch Katalysatorsysteme auf Basis von Kupfer-Chromoxid geeignet. Besonders bevorzugte Katalysatorsystem umfassen Kupfer und/oder Zink als aktive Komponente, die auf ein Trägermaterial aufgebracht sind. Als Trägermaterial eignen sich poröses Siliciumoxid und/oder Aluminiumoxid.

Der zur Hydrierung benötigte Wasserstoff kann direkt als Einsatzstoff bereitgestellt werden. Möglich ist es auch, den Wasserstoff dadurch bereitzustellen, dass ein Wasserstoff-haltiges Gas vorher in einer für den Fachmann bekannten Weise in seine Komponenten aufgetrennt wird und der Wasserstoff zur Hydrierzone geführt wird. Der Wasserstoff kann dabei weiterhin einen gewissen Anteil an Kohlenmonoxid oder anderen Gasen enthalten, weil eine vollständige Auftrennung kaum zu realisieren ist.

Das Alkoholgemisch aus der Hydrierung in Schritt e wird im nachfolgenden Schritt f in mindestens einem Trennverfahrensschritt ausgewählt aus der Gruppe, bestehend aus einer thermischen Trennung, beispielsweise Destillation, einer Extraktion, einer Kristallisation oder einer weiteren Membrantrennung, unterworfen, um den in Schritt e gebildeten Zielalkohol vom restlichen Alkoholgemisch abzutrennen. Das Trennverfahren ist vorzugsweise eine Destillation. Die entsprechenden Verfahrensbedingungen sind dem Fachmann bekannt. Es kann auch eine mehrstufige Destillation durchgeführt werden.

Zudem kann der in dem mindestens einen Trennverfahrensschritt abgetrennte Alkohol, der bei der Alkoxycarbonylierung eingesetzt wurde, zur Reaktionszone zurückgeführt werden. Bei der Rückführung kann ein Purgestrom entnommen werde, um beispielsweise Nebenprodukte der Hydrierung inerte Alkane, Aldehyde, Acetale, Ether, Ketone oder Carbons aus dem Verfahren auszuschleusen.

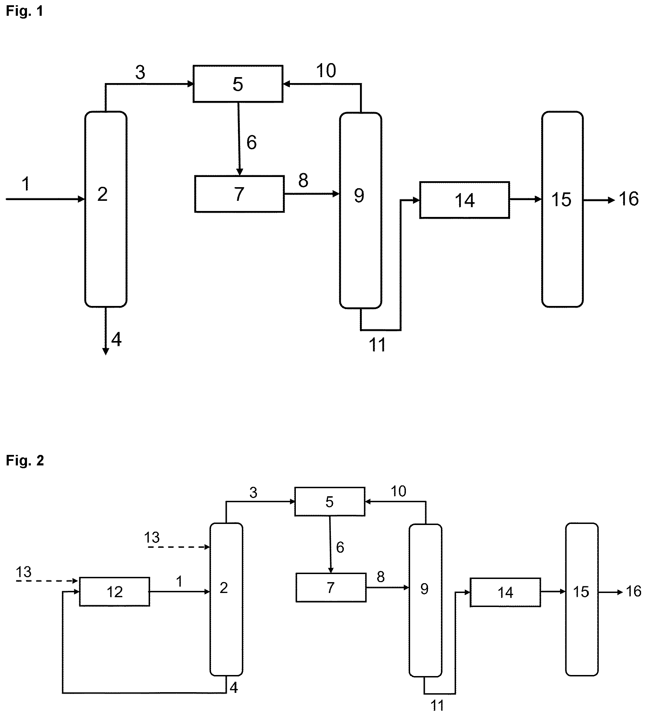

Die vorliegende Erfindung wird anhand der Abbildungen Fig. 1 und Fig. 2 näher erläutert. Die Zeichnungen zeigen spezielle Ausführungsformen und sollen den Erfindungsgegenstand nicht beschränken.

Fig. 1 zeigt eine Ausführungsform, bei der der Di-isobutenstrom (1) zur Destillationskolonne (2) in Schritt a geführt wird. Der Kopfstrom (3) ist gegenüber dem eingesetzten Di-isobutenstrom an 2,4,4-Trimethylpent-1-en angereichert. Bei der Destillation fällt außerdem der Reststrom (4) an, der gegenüber dem eingesetzten Di-isobutenstrom an 2,4,4-Trimethylpent-1-en abgereichert ist. Der Kopfstrom (3) wird nachfolgend zur Alkoxycarbonylierung (5) in Schritt b geführt, wodurch ein Produktgemisch (6) entsteht. Dieses Produktgemisch (6) durch die Katalysatorabtrennung (7) in Schritt c zum Rohproduktgemisch (8), welches in Schritt d einer Destillation (9) unterworfen wird. Dort fällt das Produkt (11) sowie die nicht umgesetzten Alkohole und das nicht umgesetzte 2,4,4-Trimethylpent-2-en und 2,4,4-Trimethylpent-1-en als Strom (10), welches zur Alkoxycarbonylierung (5) zurückgeführt wird. Das Produkt, also der 3,5,5-Trimethylhesansäurealkylester, wird dann zur Hydrierung (14) gefahren und dort zum Zielalkohol umgewandelt, die in der nachgeschalteten destillativen Auftrennung (15) als Strom (16) abgetrennt wird.

Fig. 2 betrifft eine weiter Ausführungsform, bei der zusätzlich eine Isomerisierung (12) vorhanden ist. Der eingesetzte Di-isobutenstrom (13) kann dabei zur Isomerisierung und/oder zur Destillation (2) geführt werden. Dies wird durch die gestrichelten Linien angedeutet. Der isomerisierte Strom (1) wird dann der Zulauf zur Destillation (2). Die Isomerisierung wird zudem mit dem Reststrom (4) aus der Destillation gespeist, um dort 2,4,4-Trimethylpent-2-en zumindest teilweise in 2,4,4-Trimethylpent-1-en umzuwandeln.

Die Erfindung wird nachfolgend anhand von Beispielen beschrieben. Diese dienen der Erläuterung und stellen keine Einschränkung des Erfindungsgegenstands dar.

## Patentansprüche

1. Verfahren zur Herstellung eines Zielalkohols, wobei das Verfahren zumindest die folgenden Schritte umfasst:
a. Zuführen eines Di-isobutenstroms, der 2,4,4-Trimethylpent-2-en und 2,4,4-Trimethylpent-1-en enthält, zu mindestens einer Destillationskolonne und Auftrennen des Di-isobutenstroms in mindestens einen gegenüber dem eingesetzten Di-isobutenstrom an 2,4,4-Trimethylpent-1-en angereicherten Kopfstrom, der mehr als 85 Gew.-% an 2,4,4-Trimethylpent-1-en enthält, und einen Reststrom, der gegenüber dem eingesetzten Di-isobutenstrom an 2,4,4-Trimethylpent-1-en abgereichert ist;
b. Zuführen des Kopfstroms zu einer Alkoxycarbonylierung, wodurch 2,4,4-Trimethylpent-2-en und 2,4,4-Trimethylpent-1-en mit einem Alkohol und Kohlenmonoxid in Gegenwart eines homogenen Katalysatorsystems, welches zumindest ein Metall der Gruppe 8 bis 10 des Periodensystems der Elemente oder einer Verbindung davon, einen phosphorhaltigen Liganden und eine Säure umfasst, in einer Reaktionszone unter Erhalt eines flüssigen Produktgemisches umgesetzt werden, wobei das flüssige Produktgemisch zumindest den durch die Alkoxycarbonylierung gebildeten Ester, das homogene Katalysatorsystem, nicht umgesetztes 2,4,4-Trimethylpent-2-en und 2,4,4-Trimethylpent-1-en und nicht umgesetzten Alkohol umfasst;
c. Abtrennen des homogenen Katalysatorsystems aus dem flüssigen Produktgemisch unter Erhalt eines Rohproduktgemisches, welches zumindest den durch die Alkoxycarbonylierung gebildeten Ester, nicht umgesetztes 2,4,4-Trimethylpent-2-en und 2,4,4-Trimethylpent-1-en und nicht umgesetzte Alkohole umfasst;
d. Destillative Aufarbeitung des Rohproduktgemisches in mindestens einer Destillationskolonne zur Abtrennung der nicht umgesetzten Alkohole und das nicht umgesetzte 2,4,4-Trimethylpent-2-en und 2,4,4-Trimethylpent-1-en, wobei nicht umgesetzter Alkohol und das nicht umgesetzte 2,4,4-Trimethylpent-2-en und 2,4,4-Trimethylpent-1-en abgetrennt und zur Alkoxycarbonylierung in Schritt b zurückgeführt wird;
e. Hydrieren des in Schritt d erhaltenen Esters mit Wasserstoff in Gegenwart des heterogenen Katalysatorsystems in einer Hydrierzone unter Erhalt eines Alkoholgemisches, welches zumindest den Zielalkohol, den abgespaltenen Alkohol und nicht umgesetzte Ester umfasst; und
f. Abtrennen des in Schritt e gebildeten Zielalkohols in mindestens einem Trennverfahrensschritt, ausgewählt aus thermischer Trennung, beispielsweise Destillation, Extraktion, Kristallisation und Membrantrennung.

2. Verfahren nach einem der vorhergehenden Ansprüche, wobei der Reststrom in Schritt a als Sumpfstrom oder als Seitenstrom an der mindestens einen Destillationskolonne abgenommen wird.

3. Verfahren nach einem der vorhergehenden Ansprüche, wobei der Reststrom zu einer Isomerisierung geführt wird, bei der 2,4,4-Trimethylpent-2-en unter Verwendung eines heterogenen Katalysators auf Basis eines Zeoliths oder eines Ionenaustauscherharzes zumindest teilweise zu 2,4,4-Trimethylpent-1-en isomerisiert wird.

4. Verfahren nach Anspruch 3, wobei aus der Isomerisierung ein Isomerisierungsstrom erhalten wird, in dem der Anteil des 2,4,4-Trimethylpent-2-ens geringer und der Anteil des 2,4,4-Trimethylpent-1- höher ist als im Reststrom.

5. Verfahren nach Anspruch 3 oder 4, wobei der Isomerisierungsstrom zumindest teilweise zur Destillation in Schritt a geführt wird.

6. Verfahren nach einem der Ansprüche 3 bis 5, wobei ein Einsatzstrom, mit dem das Verfahren gespeist wird und der 2,4,4-Trimethylpent-2-en und 2,4,4-Trimethylpent-1-en enthält, zusätzlich zum Isomerisierungsstrom zur Destillationseinheit in Schritt a und/oder zusätzlich zum Reststrom zur Isomerisierung geführt wird.

7. Verfahren nach einem der Ansprüche 3 bis 6, wobei die Isomerisierung bei einer Temperatur von 25 bis 90 °C, vorzugsweise 30 bis 80 °C, besonders bevorzugt 35 bis 70 °C durchgeführt wird.

8. Verfahren nach einem der Ansprüche 3 bis 7, wobei das Zeolith ein Si : Al Verhältnis im Bereich von 40 : 1 bis 200 : 1 aufweist.

9. Verfahren nach einem der Ansprüche 3 bis 8, wobei das Zeolith aus der Gruppe der beta- und gamma-Zeolithe ausgewählt wird.

10. Verfahren nach einem der Ansprüche 3 bis 7, wobei als lonenaustauscherharz solche eingesetzt werden, die durch die Sulfonierung von Phenol/Aldehyd-Kondensaten oder durch die Sulfonierung von Copolymeren von aromatischen Vinylverbindungen hergestellt werden.

11. Verfahren nach einem der vorhergehenden Ansprüche, wobei die Alkoxycarbonylierung in Schritt b bei einer Temperatur von 60 bis 120 °C, vorzugsweise 65 bis 110 °C, besonders bevorzugt 70 bis 100 °C durchgeführt wird.

12. Verfahren nach einem der vorhergehenden Ansprüche, wobei die Alkoxycarbonylierung in Schritt b bei dem Kohlenmonoxiddruck von 10 bis 35 bar, vorzugsweise 12,5 bis 30 bar, besonders bevorzugt 15 bis 25 bar durchgeführt wird.

13. Verfahren nach einem der vorhergehenden Ansprüche, wobei der Alkohol ein einwertiger Alkohol mit 1 bis 4 Kohlenstoffatomen, insbesondere Methanol, Ethanol, Propanol oder Butanol ist.

14. Verfahren nach einem der vorhergehenden Ansprüche, wobei die Abtrennung des homogenen Katalysatorsystems in Schritt c mittels Membrantrennung erfolgt.

15. Verfahren nach einem der vorhergehenden Ansprüche, wobei der Di-isobutenstrom, der Alkohol und das homogene Katalysatorsystem, zunächst in einem Mischbehälter vermischt werden, bevor sie in die Reaktionszone geleitet werden.
